# EUROPEAN PATENT SPECIFICATION

(11) **EP 4 382 141 B1**
(45) Date of publication and mention of the grant of the patent: **09.07.2025**
(21) Application number: 23208365.9
(22) Date of filing: 07.11.2023
(51) Int. Cl.: A61L 24/00, A61L 24/02, A61P 19/02, A61P 19/10

(54) **COMPOSITE BIOCEMENT SYSTEM**
BIOZEMENT-KOMPOSIT-SYSTEM
SYSTÈME DE BIOCIMENT COMPOSITE

(30) Priority: 05.12.2022 SK 1172022
(43) Date of publication of application: 12.06.2024
(73) Proprietor: Ustav materialoveho vyskumu Slovenskej Akademie vied, verejna vyskumna institucia, 040 01 Kosice (SK)
(72) Inventor: Medvecky, Lubomir, 040 13 Kosice (SK); Giretova, Maria, 040 01 Kosice (SK); Stulajterova, Radoslava, 040 11 Kosice (SK); Kresakova, Lenka, 044 58 Sena (SK); Vdoviakova, Katarina, 053 33 Nalepkovo (SK)
(74) Representative: Majlingová, Zuzana

(56) References cited:
- SK-B6- 288 348
- BIGHAM-SADEGH AMIN ET AL: "Effects of Honey and Hydroxyapatite on Bone Healing in Rats", TRAUMA MONTHLY, vol. 23, no. 4, 30 June 2018 (2018-06-30), XP093146664, ISSN: 2251-7464, DOI: 10.5812/traumamon.56119

## Description

### Technical field

The invention relates to the composition and use of composite biocement systems for the regeneration and reconstruction of hard tissue, cartilage defects and osteochondral defects in medical applications.

### Background Art

The clinical application of calcium phosphate systems in medicine has been known since the identification of the bone mineral composition but autologous and allogeneic bone implants have been and still are preferentially used, with undeniable advantages such as their osteoinductivity, bioactivity, biocompatibility, osteoconductivity, as well as a high degree of ossteointegration with the surrounding tissue after healing of the damaged sites. The above characteristics have not been observed in such complexity in synthetic types of calcium phosphate biomaterials especially in the case of ossteoinduction and ossteointegration. The advantage of synthetic biomaterials is that there is no immunogenic response and possible transmission of diseases from the donor. Moreover, there are no complications associated with autologous transplantation with the removal of biological material from another part of the body. The widespread use of calcium phosphate biomaterials for commercial purposes in the form of coatings, hydroxyapatite ceramic blocks and fixtures begins in the 1990s, while porous ceramic scaffolds were only developed at the end of the 20th century (M. Jarcho, M. Clin. Orthop. Relat. Res. 157 (1981) 259; H. A. Aoki et al., J. Dent. Eng. 18 (1977) 86-89). Calcium phosphate biocements (CPC) are biocompatible, osteoinductive and osteoconductive materials depending on composition and they are used for reconstruction and regeneration of bone tissues in the body, because due to their easy application in the form of a moldable paste, they are predestined for perfect filling of various types of defects in bone tissues in the cranio-maxillofacial region. (A. Kolk et al. Journal of Cranio-Maxillofacial Surgery 40 (2012) 706-718; F. Baino Acta Biomaterialia. 10 (8) (2014) 3372-3397), in dental medicine and orthopaedics (J. Yao, AM Ho, Operative Techniques in Orthopaedics. 19 (2) (2009) 77-87).

In terms of addressing osteochondral defects, the patented systems are mainly oriented towards the preparation of cellular types of biopolymer 3D-substrates with seeded autologous stem cells or chondrocytes in the form of, for example, devitalized allo- or xeno-grafts of cartilage containing cartilage growth factors (WO2009011849, WO2010083051A2), formation of synthetic matrix on substrates by chondrocytes in-vitro (WO2009106642A1), injectable biopolymer microspheres (WO210017265), cellulose hydrogels (WO2009155583A1), photocomposites based on chitosan/collagen blends (WO2010120757A2), silk-based multistructural porous systems with fibrin adhesive bonded layers (WO2011156586A2), composite calcium phosphate/biopolymer films (JP2004049626) and fibrous bilayer scaffolds (US8475531), composite implants made of decellularized cartilage and demineralized bone (MD1177), calcium phosphate/collagen sponges (CN106139255) or multiphase scaffolds (WO2017118863), gradient porous scaffolds containing PLGA microparticles and hydrogels (US2017239395), devitalized micronized extracellular matrix (US2017232144), multi-layered collagen/hyaluronic acid blends (US2017157288), double-walled structures with hydroxyapatite and fibroin (WO2016100721) chemically bonded with chitosan (CN106178126), ceramic structural bi-layered system with different porous layers (US2007113951), collagen/hydroxyapatite multilayer structures (US2009232875). In addition, papers have been published and patented on the application of calcium phosphate biocements demonstrating successful healing of bone and osteochondral defects in vivo (SK288818; L. Medvecky et al., Materials 14 (2021) 436).

CPCs are most commonly represented by two- and multi-component systems, with individual components undergoing dissolution and hydrolysis, which changes the nature of the environment (e.g., pH, increase in ion concentration) with subsequent supersaturation of surroundings in relation to the final product of precipitation, most commonly hydraxyapatite (HAP) (or its calcium-deficient forms, CDHAP) (Ch. Liu et al., J. Biomed. Mater. Res. 35 (1997) 75). Hydroxyapatite self-setting biocement types are mostly composed of tetracalcium phosphate/monetite (CaHPO₄) (TTCP/DCPA or brushite (CaHPO₄.2H₂O) mixtures or contain a high proportion of tricalcium phosphate (mostly the high-temperature α-form), and after mixing with the liquid component (phosphate solutions), a gradual transformation of the phases to CDHAPs takes place and setting of the whole system occurs (L.C. Chow et al., Biomaterials 26 (2005) 393; L.A. dos Santos et al., Biomaterials 23 (2002) 2035). The physicochemical properties in relation to the setting process of TTCP/DCPA-based CPCs are significantly influenced by the Ca/P ratio of the CPC, the addition of organic additives (carboxylic acids, alginates, phytoleic acid, chitosan, etc.), various inorganic fillers (inert oxides - SiO₂, ZrO₂, silicon nitrides, whiskers, etc.), as well as by the addition of HAP nanocrystalline particles (K.S. Tenhuisen, P.W. Brown, J. Biomed. Mater. Res. 36 (1997) 233; C.S. Liu et al., Biomaterials 24 (2003) 4103; K. Ishikawa, E.D. Eanes, J. Dent. Res. 72(2) (1993) 474; Q. Yang et al., Biomaterials 23 (2002) 2751-2760; K. Takahashi et al., Ceramics International 30 (2004) 199-203). The amorphous to nanocrystalline nature of HAP particles in CPCs influences the in vitro behaviour of biologically active bone tissue cells (osteoclasts, osteoblasts) and promotes cell proliferation and growth (T. Yuasa et al., J. Biomed. Mater. Res. 54 (2001) 344-350; C. Itthichaisri et al., J. Biomed. Mater. Res. 82A (2007) 777-787; Han Guo et al., Acta Biomaterialia 5 (2009) 268-278; P. Quinten Ruhe et al. Biomaterials 25 (2004) 2123-2132; M. Komata, H.H. Varma, Bull. Mater. Sci. 26(4) (2003) 415). A small (~1% w/w) addition of different types of organic substances (e.g., soluble starch, cyanuric acid, cyclodextrin, dextran, polyvinylpyrrolidone) to TCP cement increased the stability of the cement paste after immersion to solutions (I. Khairoun et al., Biomaterials 20 (1999) 393). The formation of the TCP/gelatine composite significantly enhanced the compressive strength of the cement (A. Bigi et al., Biomaterials 25 (2004) 2893).

The bioactivity of the CPC cement system and especially the resorbability can be increased by the addition of calcium sulphates as an additional component of the cement, however, the amount must be optimized to obtain suitable physicochemical and mechanical properties, as they alone have poor mechanical properties and a high resorption rate (A.E.S. Van Driessche et al., (eds.), New Perspectives on Mineral Nucleation and Growth, Springer International Publishing, Switzerland, 2017). Calcium sulphate (CS) is added to cements to increase the porosity of cements up to 60% w/w (I. Lodoso-Torrecilla et al., Acta Biomaterialia 119 (2021) 1-12; J.S. Wang et al., Journal of Orthopaedic Translation 6 (2016) 10-17; B.C. Yang et al., Materials 13 (2020) 3458) and improving the mechanical properties of the original CPC mixture (J.H. Lin et al., J. Med. Biol. Eng. 32(3) (2012) 201-204), which were reduced with the rise in porosity after dissolution of CS (G. Hu et al., J. Mater. Sci.: Mater. Med. 21 (2010) 627-634). The addition of calcium sulphate hemihydrate actively affected the transformation of TTCPM cement mixture and reduced the rapid pH rise after cement paste preparation (H. Guo et al., Biomed. Mater. 1 (2006) 193-197; L. Medvecky et al., Materials 2021, 14, 2137).

The bioactivity of CPCs in relation to the formation of new bone tissue can be positively influenced by the use of biologically active agents such as bone morphogenetic proteins and growth factors dosed in very small amounts (Cornelius von Wilmowsky et al., Oral Maxillofac Surg DOI 10.1007/s10006-013-0398-1), which led to a significant increase in bone formation and neovascularisation in the rabbit femoral defect model (R. Reyes et al., Int. J. Care Injured 43 (2012) 334-342). Similarly, αTCP cement acquired osteoinductivity by adding nanogram quantities of BMP2 to the cement porous granules, from which it was gradually released after implantation into the rabbit (C. H. Kroese-Deutman et al., Biomaterials 26 (2005) 1131-1138). The aforementioned findings demonstrate the effective and powerful influence of specific substances mostly produced by cells on the complete formation of bone tissue. The problem with these substances, mostly proteins, is their high cost, the possibility of a decrease in their activity after adsorption on the biomaterial due to a change in their structural configuration as well as the necessity to kept storage conditions (mostly -20°C) after processing in order to preserve their biological characteristics. The solution may lie in the search for suitable inexpensive natural products with a relatively wide range of biological characteristics covering the stimulation of osteoinductive, osteoconductive as well as anti-inflammatory or antimicrobial properties of CPCs. Examples include Aloe vera containing acermanic acid, aloein, which increased mesenchymal stem cell (MSC) proliferation, ALP activity and promoted bone tissue growth (S. Boonyagul et al., Odontology 102 (2014) 310-317). In addition to its anti-inflammatory and antimicrobial properties, curcumin has a positive effect on the proliferation of osteoblasts and suppresses the resorption of bone tissue (A. Sahebkar et al. Pharmacol. Res. 107 (2016) 234-242). Allicin as an active component of garlic, protects osteoblasts from oxidative stress and apoptosis (L. Bayan et al., J. Phytomed. 4 (2014) 1). Green tea is another interesting example of the effective impact of a natural product on osteoporosis, inhibition of bone resorption and growth of osteoblastic activity (C.L. Shen et al., Pharmacol. Res. 64 (2011) 155-161; C. L. Shen et al., Nutr. Res. 29 (2009) 437-456).

In terms of bone tissue, bone waxes containing originally beeswax, almond oil and acetylsalicylic acid are very often applied and are characterized by a homeostatic effect in orthopaedic (total knee replacement) (H. Moo et al., J. Arthroplasty 32 (2017) 1483) or neurological surgeries because they have excellent adhesion to bone and are hydrophobic, acting as an impermeable, non-resorbable membrane that does not leak blood after their application (H. Zhou et al., J. Orthop. Transl. 17 (2019) 64-72). Their disadvantages are significant side effects associated with bacterial infection, formation of fibrous tissue, insufficient formation of new bone tissue, development of a strong inflammatory reaction, etc. The above shortcomings have been addressed by the development of new bone waxes, where beeswax has been replaced by synthetic products such as PEG, acid esters, fatty acids in conjunction with cellulose hydrogels, chitosan, gelatine, and to increase bioactivity, CPCs are added and gradual degradation of the wax was demonstrated (T. Brückner et al., Acta Biomaterialia 33 (2016) 252-263). Another type of natural product is honey, long known for its antimicrobial properties and effective effect in healing skin wounds. Lowers ambient pH, promotes vascular growth factor production, epithelialization and granulation of tissue as well as reduces swelling (A.C. Yilmaz, D. Aygin, Complementary Therapies in Medicine 51 (2020) 102388). The importance of honey for regenerative medicine applications of bone defects is still underestimated, and there are virtually no papers analysing the properties of calcium phosphate systems in ceramic or CPC form containing honey. Honey containing polyphenolic compounds with strong antioxidant properties suppresses the oxidative stress of cells, thus effectively counteracting osteoporotic changes and the decline in osteoblast activity. In animal models, it has been shown that the addition of honey to the diet increased calcium absorption into bone tissue in the acute phases and promoted biomechanical bone strength after long-term use (M. A. Kamaruzzaman et al., Evidence-Based Complementary and Alternative Medicine (2019) ID 8543618 https://doi.org/10.1155/2019/8543618). Application of honey to artificially created defects in rat mandible demonstrated significant growth of well-mineralized bone tissue already in the early stages of healing (F. Hajizadeh et al., J. Contemp Dent Pract. 19(1) (2018) 47-51). Analysis of bone tissue in a rat radius defect confirmed improved morphology of newly formed tissue in a honey/HAP composite (A. Bigham-sadegh et al., Trauma Monthly 23(4) (2018) Article ID 56119). The results of the analysis of the influence of honey on the precipitation of calcium phosphate in solutions revealed its inhibitory but also promotional effect depending on the type of honey used (S. Hidaka et al., J Periodont Res 43 (2008) 450-458).

SK 288 348 B6 discloses a cement system composed of a powder component comprising a calcium phosphate mixture of microcrystalline tetracalcium phosphate and a monetite with a Ca/ P mole ratio in the range of 1.5 to 1.68; with an average particle size of 10 to 100 nm and a liquid component comprising a solution containing up to 4% w/w of sodium dihydrogen phosphate or sodium hydrogen phosphate.

### Summary of Invention

The subject matter of the invention is the composition of a composite biocement system designed for reconstruction and regeneration of hard tissue, cartilage and osteochondral defects. The biocement system according to the invention is as defined in claims 1 and 6.

Wherein the honey in the liquid component is preferably in the range of 0.01 to 25% w/w, most preferably 5 to 20% w/w. The calcium phosphate component of the biocement is characterized by a Ca/P mole ratio corresponding to stoichiometric or calcium-deficient hydroxyapatite in the range of 1.5-1.68. The basic powdered tetracalcium phosphate component of the biocement mixture is synthesized by high temperature reaction at temperatures above 1400°C of a homogeneous powdered mixture of various calcium phosphates preferably calcium hydrogen phosphate (CaHPO₄), calcium hydrogen phosphate dihydrate (CaHPO₄.2H₂O), calcium dihydrogen phosphate (Ca(H₂PO₄)₂), calcium pyrophosphate (Ca₂P₂O₇) as well as the diammonium hydrogen phosphate ((NH₄)₂HPO₄) with calcium carbonate (CaCO₃), calcium oxide (CaO), calcium hydroxide (Ca(OH)₂) or other calcium salts which decompose at temperatures up to 1000 °C. The obtained tetracalcium phosphate is further refined by grinding in a ball mill, usually for 15-60 minutes, so that the resulting powder contains particles with a particle size of <10 µm. The final tetracalcium phosphate/monetite cement mixture is prepared in situ by reacting the synthesized milled tetracalcium phosphate with a dilute solution of orthophosphoric acid in a pure organic solvent or a mixture of solvents miscible with water preferably in ethanol, methanol, acetone, or mixtures thereof, the orthophosphoric acid being added in a well-defined amount to give a total Ca/P molar ratio in the range of 1.5-1.68. The tetracalcium forphate/monetite mixture containing calcium sulphate hemihydrate is prepared from powdered tetracalcium phosphate by in situ reaction with a mixture of orthophosphoric acid and sulfuric acid in a pure organic solvent or a mixture of solvents miscible with water and preferably in ethanol, methanol, acetone or mixtures thereof, so that the final product of the cement setting is stoichiometric hydroxyapatite or calcium-deficient forms thereof with a total Ca/P molar ratio in the range 1.5-1.68. Sulphuric acid is added in such an amount that the calcium sulphate content of the final cement mixture is preferably up to 15% w/w.

The powdered component of the composite biocement system may also be prepared directly by dry (without liquid medium) or wet homogenization of the powdered mixture of calcium phosphate precursors in a mill with a total Ca/P mole ratio in the range of 1.5-1.68, or by mechanical mixing of suspensions in water, ethanol, methanol, acetone, or mixtures thereof, which may be absolute, i.e., completely anhydrous, or may contain a well-defined amount of water in the range of up to 30 % v/v.

The dried powdered mixture does not need to be further treated in any way, and the final composite biocement system in the form of a cement paste is obtained by mixing the prepared powdered calcium phosphate mixture with a solution containing up to 4% w/w of sodium dihydrogen phosphate or sodium hydrogen phosphate or potassium dihydrogen phosphate or mixture thereof, preferably a 1-4% solution of sodium dihydrogen phosphate, sodium hydrogen phosphate, potassium dihydrogen phosphate, or a mixture thereof, and up to 25 % w/w of honey. The ratio of the powder to liquid component may be in the range of 1.5-3, preferably 2 g/ml. The setting times of biocement systems prepared according to the described method are in the range of 5-40 minutes, depending on the composition of the cement mixture, the grinding time, the concentration and the amount of liquid. The compressive strength of hardened cements is in the range up to 50 MPa, which corresponds to the strength characteristics of bone tissue. The advantage of biocement mixtures prepared by in situ reaction is a significant reduction of the pH value in the initial stages of setting below 8.4 as well as the addition of honey, which reduces the intensity of irritation, inflammatory reaction and stress factors of the surrounding tissues after application. The biocements are easy to apply at the site of the defect immediately after preparation of the cement paste and surgical repair of the defect site, have satisfactory viscosity and consistency for filling bone defects as well as high resistance to disintegration in aqueous solutions after only 2 minutes from the addition of the liquid component. In the case of cement with the addition of calcium sulphate hemihydrate, there is an increase in the concentration of calcium ions resulting from its dissolution and transformation during the setting of the cement paste. The results of in vivo experiments confirmed the formation of bone tissue of comparable morphology and composition to the original bone tissue and, in the case of an osteochondral defect, formation of a continuous hyaline cartilage with a zone structure within 12 weeks without signs of inflammatory processes at the site of the defect.

Any references in the description to methods of treatment refer to the products of the present invention for use in a method for treatment.

### Brief description of the drawings

Figure 1 shows a radiograph of a sheep limb documenting bone formation in the tibia and subchondral defect of the distal femoral epiphysis after 12 weeks of healing.
Figure 2 shows a macroscopic image of a condyle section documenting bone formation in the tibia and subchondral defect of the distal femoral epiphysis after 12 weeks of healing.

### Examples

### Example 1

A composite biocement system composed of a calcium phosphate mixture containing powdered microcrystalline tetracalcium phosphate with an average particle size <10 µm and nanocrystalline monetite. The content of the individual components in the biocement corresponds to a Ca/P mole ratio of 1.68. The liquid component of the cement contained 2% w/w of NaH₂PO₄ and 10% w/w of honey. The setting time was 5 min and the compressive strength reached 31 MPa.

### Example 2

A composite biocement system composed of a calcium phosphate mixture containing powdered microcrystalline tetracalcium phosphate with an average particle size <10 µm and nanocrystalline monetite. The content of the individual components in the biocement corresponds to a Ca/P mole ratio of 1.60. The liquid component of the cement contained 2% w/w of NaH₂PO₄ and 10% w/w of honey.

### Example 3

A composite biocement system composed of 20% w/w of tricalcium phosphate and calcium phosphate mixture with a Ca/P mole ratio of 1.68 containing powdered microcrystalline tetracalcium phosphate with an average particle size <10 µm and monetite. The liquid component of the cement contained 1% w/w of NaH₂PO₄ and 10% w/w of honey.

### Example 4

A composite biocement system consisting of tricalcium phosphate with an average particle size < 10µm. The liquid component of the cement contained 1% w/w of NaH₂PO₄ and 10% w/w of honey. The powder-to-liquid ratio was 2. The setting time was 5 min and the compressive strength reached 48 MPa.

### Example 5

A composite biocement system consisting of tricalcium phosphate with an average particle size <10 µm. The liquid component of the cement contained 5% w/w of honey dissolved in water. The powder-to-liquid ratio was 1.7.

### Example 6

A composite biocement system consisting of tricalcium phosphate with an average particle size <10 µm. The liquid component of the cement contained 2% w/w of KH₂PO₄, 1% w/w of NaH₂PO₄ and 10% w/w of honey.

### Example 7

A composite biocement system composed of 10% w/w of tricalcium phosphate and calcium phosphate mixture with a Ca/P mole ratio of 1.5 containing powdered microcrystalline tetracalcium phosphate with an average particle size <10 µm and monetite. The liquid component of the cement contained 1% w/w of NaH₂PO₄ and 5% w/w of honey.

### Example 8

A composite biocement system consisting of tricalcium phosphate with an average particle size <10 µm. The liquid component of the cement contained 2% w/w of NaH₂PO₄ and 5% w/w of honey.

### Example 9

A composite biocement system composed of 10% w/w of calcium sulphate hemihydrate and calcium phosphate mixture with a Ca/P mole ratio of 1.68 containing powdered microcrystalline tetracalcium phosphate with an average particle size <10 µm and monetite. The liquid component of the cement contained 1% w/w of NaH₂PO₄ and 5% w/w of honey.

### Example 10

A composite biocement system composed of 5% w/w of calcium sulphate hemihydrate and calcium phosphate mixture with a Ca/P mole ratio of 1.68 containing powdered microcrystalline tetracalcium phosphate with an average particle size <10 µm and monetite. The liquid component of the cement contained 2% w/w of NaH₂PO₄ and 10% w/w of honey.

### Example 11

A composite biocement system composed of 80% w/w of tricalcium phosphate and calcium phosphate mixture with a Ca/P mole ratio of 1.68 containing powdered microcrystalline tetracalcium phosphate with an average particle size <10 µm and monetite. The liquid component of the cement contained 4% w/w of NaH₂PO₄ and 10% w/w of honey.

### Example 12

A composite biocement system composed of 20% w/w of tricalcium phosphate and calcium phosphate mixture with a Ca/P mole ratio of 1.68 containing powdered microcrystalline tetracalcium phosphate with an average particle size <10 µm and monetite. The liquid component of the cement contained 2% w/w of KH₂PO₄ and 10% w/w of honey.

### Example 13

A composite biocement system composed of 20% w/w of tricalcium phosphate, 10 % w/w of calcium sulphate hemihydrate and calcium phosphate mixture with a Ca/P mole ratio of 1.68 containing powdered microcrystalline tetracalcium phosphate with an average particle size <10 µm and monetite. The liquid component of the cement contained 2% w/w of NaH₂PO₄ and 5% w/w of honey.

### Example 14

A composite biocement system composed of a calcium phosphate mixture containing powdered microcrystalline tetracalcium phosphate with an average particle size <10 µm and monetite. The content of the individual components in the biocement corresponds to a Ca/P mole ratio of 1.68. The liquid component of the cement contained 2% w/w of NaH₂PO₄ and 20% w/w of honey.

### Example 15

A composite biocement system composed of 20% w/w of tricalcium phosphate, 5 % w/w of hydroxyapatite and calcium phosphate mixture with a Ca/P mole ratio of 1.68 containing powdered microcrystalline tetracalcium phosphate with an average particle size <10 µm and monetite. The liquid component of the cement contained 2% w/w of NaH₂PO₄ and 10% w/w of honey.

### Example 16 - Method of preparation of composite biocement systems according to Examples 1, 2, and 14

The initial powdered tetracalcium phosphate phase was prepared by annealing a mixture of CaHPO₄.2H₂O and CaCO₃ so that the Ca/P mole ratio was equal to 2.0 at 1420°C for 2 hours in air. The tetracalcium phosphate phase was crushed and milled in a ball mill for 30 minutes and the resulting powder had an average particle size equal to 8 µm. The calcium phosphate cement system was prepared in situ by milling powdered tetracalcium phosphate in a planetary ball mill for 30 minutes in an amount of orthophosphoric acid solution (diluted 1:5 by volume in absolute ethanol) that corresponds to the resulting Ca/P molar ratio of Examples 1 and 2. The result was a homogeneous powder precursor mixture containing microcrystalline tetracalcium phosphate and nanocrystalline monetite, to which a liquid component is added containing 2% w/w of NaH₂PO₄ and 10% w/w of honey. The powder-to-liquid ratio was 2.

### Example 17 - Method of preparation of composite biocement systems according to Examples 9 and 10

The initial powdered tetracalcium phosphate phase was prepared by mixing CaHPO₄.2H₂O and CaCO₃ so that the Ca/P mole ratio was equal to 2.0. The mixture was then annealed at 1450°C for 4 hours in air. The tetracalcium phosphate phase was crushed, milled in a ball mill for 30 minutes and sieved (Mesh 250). Subsequently, the powdered tetracalcium phosphate was placed in a grinding vessel and milled in a planetary ball mill in a solution of a mixture of orthophosphoric acid and sulfuric acid (mixture diluted 1:10 in 80% ethanol) for 30 minutes. The amount of orthophosphoric acid and sulfuric acid was added so that the total Ca/P mole ratio in the suspension was equal to 1.68 and the calcium sulphate content was 10% w/w. The resulting suspension was poured onto a watch glass and dried at 105°C for 2 hours, and the resulting powdered biocement system was mixed with a liquid cement component containing 1% w/w of NaH₂PO₄ and 5% w/w of honey. The powder-to-liquid ratio was 2.

### Example 18 - Method of preparation of composite biocement systems according to Example 13

The powdered tetracalcium phosphate/monetite/calcium sulphate hemihydrate cement mixture prepared according to Example 17 was mixed with tricalcium phosphate so that the content of the tricalcium phosphate component in the biocement system was 20% w/w, and the resulting powdered biocement system was mixed with a liquid cement component comprising 2% w/w of KH₂PO₄ and 10% w/w of honey. The powder-to-liquid ratio was 2.

### Example 19 - Method of preparation of composite biocement systems according to Examples 3, 7, 11, and 12

The powdered tetracalcium phosphate phase was prepared by mixing CaHPO₄ and CaCO₃ so that the Ca/P mole ratio was equal to 1.5. The mixture was then annealed at 1320°C for 2 hours in air. The tricalcium phosphate phase was crushed, milled in a ball mill for 30 minutes and sieved (Mesh 250). The powdered tetracalcium phosphate/monetite calcium phosphate mixture prepared according to Examples 1 and 2 was mixed with tricalcium phosphate so that the content of the tricalcium phosphate component in the biocement system was 20% w/w, and the resulting powdered biocement system was mixed with a liquid cement component comprising 2% w/w of KH₂PO₄ and 10% w/w of honey. The powder-to-liquid ratio was 2.

### Example 20 - Method for the preparation of composite biocement systems according to Example 15

The powdered tetracalcium phosphate/monetite calcium phosphate mixture prepared according to Example 19 was mixed with tricalcium phosphate and hydroxyapatite so that the content of the tricalcium phosphate component in the biocement system was 20% w/w and the content of the hydroxyapatite component was 5% w/w, and the resulting powdered biocement system was mixed with a liquid cement component comprising 2% w/w of KH₂PO₄ and 10% w/w of honey. The powder-to-liquid ratio was 2.

### Example 21 - Method of preparation of composite biocement systems according to Examples 4, 5, 6, and 8

The tricalcium phosphate phase was crushed, milled in a ball mill for 30 minutes, sieved (Mesh 250) and mixed with a liquid cement component containing 2% w/w of KH₂PO₄, 1% w/w of Na₂HPO₄ and 10% w/w of honey. The powder-to-liquid ratio was 2.5.

### Example 22

A subchondral articular cartilage defect in the left knee joint was induced in female sheep aged 1.5-2 years under general anaesthesia. The incision was made from the left lateral side, from the medial patellar ligament distal to the tibial tuberosity. The knee joint was made accessible over the weight-bearing surface of the medial femoral condyle. The subcutaneous ligament and superficial fascia were disrupted. In flexion of the knee joint and partial luxation of the knee bone, the Osteochondral autograft transfer system (Arthrex) kit was used to induce an articular cartilage defect at a predefined site of the distal femoral epiphysis (distal femoral epiphysis - 1x left medial condyle), with a diameter of 8 mm and a depth of 10 mm. The site of the formed defect was subsequently filled with calcium phosphate biocement according to Example 1.

### Example 23

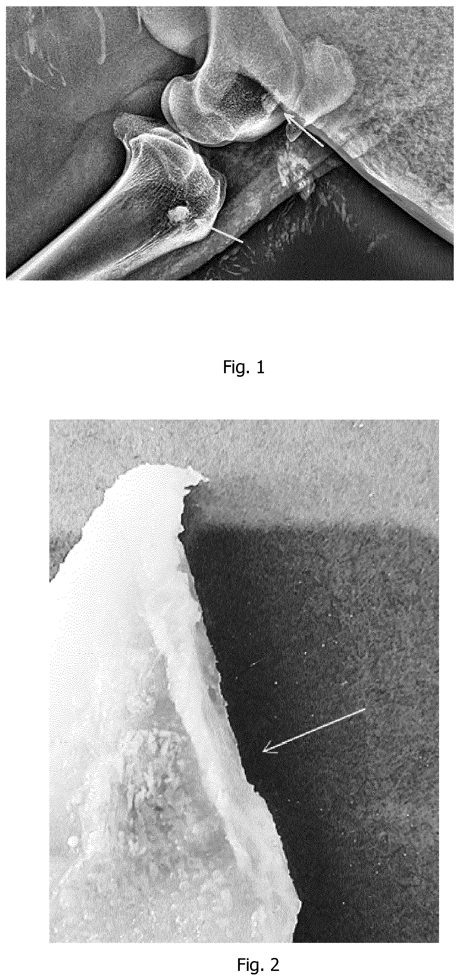

A subchondral articular cartilage defect in the left knee joint was induced in female sheep aged 1.5-2 years under general anaesthesia. The incision was made from the left lateral side, from the medial patellar ligament distal to the tibial tuberosity. The knee joint was made accessible over the weight-bearing surface of the medial femoral condyle. The subcutaneous ligament and superficial fascia were disrupted. In flexion of the knee joint and partial luxation of the knee bone, the Osteochondral autograft transfer system (Arthrex) kit was used to induce an articular cartilage defect at a predefined site of the distal femoral epiphysis (distal femoral epiphysis - 1x left medial condyle) and the proximal part of the tibia, with a diameter of 8 mm and a depth of 10 mm. The site of the formed defect was subsequently filled with calcium phosphate biocement according to Example 5. Sampling was performed 3 months after implantation of the tested biomaterial. Histological analyses showed no inflammatory process at the implantation site, morphological evaluation demonstrated the formation of hyaline cartilage tissue with a zonal structure, and histochemical analysis confirmed the presence of glycosaminoglycans in active chondrocytes. Macrographs and radiographs confirmed almost complete healing of the tibial and subchondral defect (Fig. 1, Fig. 2).

### Evaluation

The advantage of biocement mixtures prepared by in situ reaction is a significant reduction of the pH value in the initial stages of solidification below 8.4 as well as the addition of honey, reduces the intensity of irritation, inflammatory reaction and stress factors of the surrounding tissues after application. An in vitro comparison of the total antioxidant capacity of extracts from the composite biocement system and cement without the addition of honey demonstrated more than twice the antioxidant capacity of the composite systems. Similarly, statistically significantly increased (20-120%) osteogenic gene expression of osteocalcin, osteonectin and osteopontin was identified after in vitro culture of osteoblasts in extracts of composite systems compared to extracts of pure cement.

Fig. 1 and 2 show complete formation of compact and subchondral bone on the surface of the original defect site with significant resorption and remodeling of the biocement also in the area of spongiosis and the formation of a compact layer of hyaline cartilage well interconnected with the newly formed subchondral bone tissue.

## Claims

1. A composite biocement system **characterized in that** it is composed of a powder component comprising a calcium phosphate mixture of microcrystalline tetracalcium phosphate with an average particle size of less than 10µm and a monetite with a Ca/P mole ratio in the range of 1.5 to 1.68 and a liquid component comprising honey dissolved in a solution containing up to 4% w/w of sodium dihydrogen phosphate or sodium hydrogen phosphate or potassium dihydrogen phosphate or mixture thereof.

2. The composite biocement system according to claim 1, **characterized in that** the liquid component comprises up to 25% w/w of honey.

3. The composite biocement system according to claim 1, **characterized in that** it comprises at least one powder component selected from tricalcium phosphate, hydroxyapatite and calcium sulphate hemihydrate.

4. The composite biocement system according to claim 2, **characterized in that** it comprises up to 15% w/w of calcium sulphate hemihydrate.

5. The composite biocement system according to claim 2, **characterized in that** it comprises up to 90% w/w of tricalcium phosphate and up to 10% w/w of hydroxyapatite.

6. A composite biocement system, **characterized in that** it is composed of powdered tricalcium phosphate component with an average particle size of less than 10µm and a a liquid component comprising honey dissolved in a solution containing up to 4% w/w of sodium dihydrogen phosphate or sodium hydrogen phosphate or potassium dihydrogen phosphate or mixture thereof, wherein the liquid component comprises up to 25% w/w of honey.

7. A method for preparing the composite biocement system according to claim 1, **characterized in that** the powdered tetracalcium phosphate phase is milled within 60 minutes in a solution of orthophosphoric acid diluted 1:5 to 1:10 by volume in one or mixtures of organic solvents such as ethanol, methanol, acetone with a water content of up to 30% v/v so that the Ca/P ratio in the final mixture is in the range of 1.5 to 1.68 and the liquid component is prepared by dissolving honey in a solution containing up to 4% w/w of sodium dihydrogen phosphate or sodium hydrogen phosphate or potassium dihydrogen phosphate or a mixture thereof.

8. The method for preparing the composite biocement system according to claim 2, **characterized in that** the powdered tetracalcium phosphate phase is milled in a dilute solution of orthophosphoric acid and sulfuric acid in an amount such that the total Ca/P molar ratio is in the range of 1.5 to 1.68 and the calcium sulphate hemihydrate content is up to 15% w/w, and dilution in an organic solvent or mixtures of organic solvents miscible with water and containing up to 30% w/w of water was in a ratio of 1:5 to 1:10, the milling time was up to 60 minutes and the mixture obtained was dried at a temperature of up to 105°C or in vacuum and the liquid component was prepared by dissolving the honey in a solution containing up to 4% w/w of sodium dihydrogen phosphate or sodium hydrogen phosphate or potassium dihydrogen phosphate or a mixture thereof.

9. The composite biocement system according to claims 1 to 5, for use at the site of a bone, cartilage or osteochondral defect during surgical repair, reconstruction or replacement of bone or cartilage tissue.

## Patentansprüche

1. Ein Biozement-Verbundsystem, **dadurch gekennzeichnet, dass** es aus einer Pulverkomponente, die eine Calciumphosphatmischung aus mikrokristallinem Tetracalciumphosphat mit einer durchschnittlichen Teilchengröße von weniger als 10 µm und einem Monetit mit einem Ca/P-Molverhältnis im Bereich von 1,5 bis 1,68 umfasst, und einer flüssigen Komponente, die Honig umfasst, der in einer Lösung gelöst ist, die bis zu 4 Gew.-% Natriumdihydrogenphosphat oder Natriumhydrogenphosphat oder Kaliumdihydrogenphosphat oder eine Mischung davon enthält, besteht.

2. Das Biozement-Verbundsystem nach Anspruch 1, **dadurch gekennzeichnet, dass** die flüssige Komponente bis zu 25 Gew.-% Honig umfasst.

3. Das Biozement-Verbundsystem nach Anspruch 1, **dadurch gekennzeichnet, dass** es mindestens eine Pulverkomponente umfasst, die aus Tricalciumphosphat, Hydroxyapatit und Calciumsulfat-Halbhydrat ausgewählt ist.

4. Das Biozement-Verbundsystem nach Anspruch 2, **dadurch gekennzeichnet, dass** es bis zu 15 Gew.-% Calciumsulfat-Halbhydrat umfasst.

5. Das Biozement-Verbundsystem nach Anspruch 2, **dadurch gekennzeichnet, dass** es bis zu 90 Gew.-% Tricalciumphosphat und bis zu 10 Gew.-% Hydroxylapatit umfasst.

6. Das Biozement-Verbundsystem, **dadurch gekennzeichnet, dass** es aus einer pulverförmigen Tricalciumphosphatkomponente mit einer durchschnittlichen Teilchengröße von weniger als 10 µm und einer flüssigen Komponente, die Honig umfasst, der in einer Lösung gelöst ist, die bis zu 4 Gew.-% Natriumdihydrogenphosphat oder Natriumhydrogenphosphat oder Kaliumdihydrogenphosphat oder eine Mischung davon enthält, wobei die flüssige Komponente bis zu 25 Gew.-% Honig umfasst, besteht.

7. Verfahren zur Herstellung des Biozement-Verbundsystems nach Anspruch 1, **dadurch gekennzeichnet, dass** die pulverisierte Tetracalciumphosphatphase innerhalb von 60 Minuten in einer Lösung aus Orthophosphorsäure, verdünnt im Volumenverhältnis 1:5 bis 1:10 in einem organischen Lösungsmittel oder einer Mischung organischer Lösungsmittel wie Ethanol, Methanol, Aceton mit einem Wassergehalt von bis zu 30 % Vol.-% gemahlen ist, so dass das Ca/P-Verhältnis in der Endmischung im Bereich von 1,5 bis 1,68 liegt und die flüssige Komponente durch Auflösen von Honig in einer Lösung hergestellt wird, die bis zu 4 Gew.-% Natriumdihydrogenphosphat oder Natriumhydrogenphosphat oder Kaliumdihydrogenphosphat oder eine Mischung davon enthält.

8. Verfahren zur Herstellung des Biozement-Verbundsystems nach Anspruch 2, **dadurch gekennzeichnet, dass** die pulverisierte Tetracalciumphosphatphase in einer verdünnten Lösung aus Orthophosphorsäure und Schwefelsäure in einer solchen Menge gemahlen wird, dass das Gesamtmolverhältnis Ca/P im Bereich von 1,5 bis 1,68 liegt und der Calciumsulfat-Halbhydrat-Gehalt bis zu 15 Gew.-% beträgt, und die Verdünnung in einem organischen Lösungsmittel oder Gemischen organischer Lösungsmittel, die mit Wasser mischbar sind und bis zu 30 Gew.-% Wasser enthalten, im Verhältnis 1:5 bis 1:10 erfolgt, die Mahlzeit bis zu 60 Minuten betrug und die erhaltene Mischung bei einer Temperatur von bis zu 105°C oder im Vakuum getrocknet wurde und die flüssige Komponente durch Auflösen des Honigs in einer Lösung hergestellt wurde, die bis zu 4 Gew.-% Natriumdihydrogenphosphat oder Natriumhydrogenphosphat oder Kaliumdihydrogenphosphat oder eine Mischung davon enthielt.

9. Das Biozement-Verbundsystem nach den Ansprüchen 1 bis 5, zur Verwendung an der Stelle eines Knochen-, Knorpel- oder osteochondralen Defekts während der chirurgischen Reparatur, Rekonstruktion oder des Ersatzes von Knochen- oder Knorpelgewebe.

## Revendications

1. Un système de biociment composite **caractérisé par le fait qu'il** est composé d'un composant en poudre comprenant un mélange de phosphate de calcium de phosphate tétracalcique microcristallin avec une taille moyenne de particules inférieure à 10µm et une monétite avec un rapport molaire Ca/P dans la fourchette de 1,5 à 1,68 et d'un composant liquide comprenant du miel dissous dans une solution contenant jusqu'à 4 % p/p de dihydrogénophosphate de sodium ou d'hydrogénophosphate de sodium ou de dihydrogénophosphate de potassium ou d'un mélange de ceux-ci.

2. Le biociment composite selon la revendication 1, **caractérisé par le fait que** le composant liquide contient jusqu'à 25 % p/p de miel.

3. Le biociment composite selon la revendication 1, **caractérisé par le fait qu'il** comprend au moins un composant en poudre sélectionné parmi phosphate tricalcique, hydroxyapatite et sulfate de calcium hémihydraté.

4. Le biociment composite selon la revendication 2, **caractérisé par le fait qu'il** comprend jusqu'à 15 % p/p de sulfate de calcium hémihydraté.

5. Le biociment composite selon la revendication 2, **caractérisé par le fait qu'il** comprend jusqu'à 90 % p/p de sulfate de calcium hémihydraté et jusqu'à 10 % p/p d'hydroxyapatite.

6. Le système de biociment composite **caractérisé par le fait qu'il** est composé d'un composant en poudre de phosphate tricalcique avec une taille moyenne de particules inférieure à 10µm et d'un composant liquide comprenant du miel dissous dans une solution contenant jusqu'à 4 % p/p de dihydrogénophosphate de sodium ou d'hydrogénophosphate de sodium ou de dihydrogénophosphate de potassium ou d'un mélange de ceux-ci, où le composant liquide contient jusqu'à 25 % p/p de miel.

7. Procédé pour la préparation du système de biociment composite selon la revendication 1, **caractérisé par le fait que** la phase de phosphate tétracalcique en poudre est broyée dans les 60 minutes dans une solution d'acide orthophosphorique diluée dans une proportion de 1:5 à 1:10 en volume dans un solvant organique ou dans des mélanges de solvants organiques tels que l'éthanol, le méthanol, l'acétone avec une teneur en eau allant jusqu'à 30 % v/v, de sorte que le rapport Ca/P dans le mélange final soit compris entre 1,5 et 1,68 et le composant liquide soit préparé en dissolvant le miel dans une solution contenant jusqu'à 4 % p/p de dihydrogénophosphate de sodium ou d'hydrogénophosphate de sodium ou de dihydrogénophosphate de potassium ou d'un mélange de ceux-ci.

8. Procédé pour la préparation du système de biociment composite selon la revendication 2, **caractérisé par le fait que** la phase de phosphate tétracalcique en poudre est broyée dans une solution diluée d'acide orthophosphorique et acide sulfurique dans une quantité telle que le rapport molaire Ca/P total soit compris entre 1,5 et 1,68 et la teneur en sulfate de calcium hémihydraté soit jusqu'à 15 % p/p, où la dilution dans un solvant organique ou des mélanges de solvants organiques miscibles avec l'eau et contenant jusqu'à 30 % p/p d'eau est dans un rapport de 1:5 à 1:10, le temps de broyage est jusqu'à 60 minutes et le mélange obtenu est séché à une température de jusqu'à 105°C ou sous vide, et le composant liquide est préparé en dissolvant le miel dans une solution contenant jusqu'à 4 % p/p de dihydrogénophosphate de sodium ou d'hydrogénophosphate de sodium ou de dihydrogénophosphate de potassium ou d'un mélange de ceux-ci.

9. Le système de biociment composite selon les revendications de 1 à 5 pour une utilisation sur le site d'un défaut osseux, cartilagineux ou ostéochondral lors d'une réparation chirurgicale, d'une reconstruction ou d'un remplacement du tissu osseux ou cartilagineux.
